# EUROPEAN PATENT APPLICATION

(11) **EP 4 524 566 A1**
(43) Date of publication of application: **19.03.2025**
(21) Application number: 24199412.8
(22) Date of filing: 10.09.2024
(51) Int. Cl.: G01N 33/543

(54) **DETECTING ANTIBODIES USING ANTIGENS**

(30) Priority: 12.09.2023 US 202363538042 P; 05.09.2024 US 202418825426
(71) Applicant: Inova Diagnostics, Inc., San Diego, CA 92131 (US)
(72) Inventor: Mahler, Michael, San Diego, 92131 (US); Bentow, Chelsae, San Diego, 92131 (US)
(74) Representative: Müller-Boré & Partner Patentanwälte PartG mbB

(57) **Abstract**

An example complex includes a solid support and at least two different types of antigens attached to the solid support. The complex includes a carrier protein attached to the solid support. At least one of the different types of antigens is attached to the carrier protein. The at least one of the different types of antigens is attached to the solid support through the carrier protein.

## Description

### CROSS-REFERENCE TO RELATED APPLICATION

This application claims the benefit of, and priority to, U.S. Provisional Application No. 63/538,042, which is tilted "Detecting Antibodies Using Antigens", and which was filed on September 12, 2023. U.S. Provisional Application No. 63/538,042 is incorporated by reference into this application.

### TECHNICAL FIELD

This specification relates detecting antibodies using antigens.

### BACKGROUND

An antinuclear antibody (ANA) is a type of autoantibody that binds to the content of a cell nucleus and is a defining feature of autoimmune connective tissue disease. An example of an ANA test is a test that detects ANAs in biological fluids, such as blood, or bodily tissue of a subject. In some cases, when using the indirect immunofluorescence (IIF) test using HEp-2 cells, autoantibodies targeting cytoplasmic antigens such as Jo-1 or ribosomal P proteins are detected and reported. Therefore it was recommended to change the nomenclature to anti-cellular antibodies or "ACA". However, the term ANA persists in the literature and, therefore, ANA and ACA are used interchangeably herein.

A positive ANA test result means that autoantibodies are present in a subject, which may be a sign of disease or other condition. Multi-analyte assay/testing systems that test for multiple types of ANAs use a single antigen immobilized on a single solid support, such as a particle or a bead. An antibody in a sample binds to the antigen and the result is detected based, for example, on fluorescence of the bound antigen/antibody. The amount of fluorescence detected is directly proportional to the amount of antibodies contained in the sample. A relatively large amount of antibodies indicates a higher likelihood of a disease that is associated with target antigens.

Some multi-analyte assay/testing systems of the type described above do not include sufficient antigens to capture sufficient antibodies in comparison to the IIF assay on a cell substrate (e.g. HEp-2 cells), making detection of diseases and other conditions challenging in some cases. In addition, depending on the disease or condition, multiple beads are used in these assays which can lead to inefficient workflows since each bead needs to be detected and the combined signals analyzed to determine the disease or condition. Calibration with multiple beads also can take a lot of time and the analysis can require complex computational assistance as well as a high degree of skill from a technician.

### SUMMARY

An example complex includes a solid support and at least two different types of antigens attached to the solid support. The complex may include one or more of the following features, either alone or in combination.

The complex may include a carrier attached to the solid support;. A least one of the different types of antigens may be attached to the carrier. At least one of the different types of antigens may be attached to the solid support through the carrier. The complex may include a linker to attach at least one of the different types of antigens to the carrier. The linker may include a polyethylene glycol (PEG) spacer or biotin.

The carrier may include at least one of streptavidin, a polymerized protein, polymerized streptavidin with biotin, bovine serum albumin (BSA), human serum albumin (HSA), polyethylene glycol (PEG) spacer attachment, or adipic acid dihydrazide (ADH).

The complex may include multiple carriers. Each of the carriers may be attached to the solid support. Each of the different types of antigens may be attached to a respective one of the multiple carriers. Each of the different types of antigens may be attached to the solid support through the respective one of the multiple carriers.

The complex may include a carrier attached to the solid support. Each of the different types of antigens may be attached to the carrier. Each of the different types of antigens may be attached to the solid support through the carrier.

At least two different types of antigens may include a group of markers for a disease or condition of a subject. At least one of the different types of antigens may be part of a cellular extract that is attached to the solid support. A first one of the different types of antigens may include a purified antigen and a second one of the different types of antigens may be part of a cellular extract.

The at least two different types of antigens may include at least two of the following: Scl-70, centromere, dsDNA, Ro52, Ro60, SS-B, Ribo-P, DFS-70, EJ, Sp100, Gp210, actin, AMA-M2, AMA-M2/MIT3, TIF1-γ, NXP-2, SAE, PL-7, PL-12, OJ, HMGCR, SRP, Mi-2, MDA-5, RNA Helicase, Ki/SL, Sm/RNP, SS-A, PM/Scl, RNA Pol III, Th/To, Fibrillarin, Sm, Ku, Jo-1, RNP, BICD2, HK, KL, LC1, LKM-1, SLA, SS-A/Ro52, Scl-70, Ku, Centromere proteins, U11/U12 RNP, Hexokinase-1, or Kelch-12.

At least one of the different types of antigens may bind to an antinuclear antibody (ANA). The complex may include a label associated with the solid support. The label may be associated with a disease, condition, or analyte that is detectable using the at least two different antigens.

An example kit includes a first solid support including first antigens attached thereto, with the first antigens including at least two different types of antigens; and a second solid support including at least one second antigen attached thereto, with the at least one second antigen being different than each of the first antigens. One or more of the first antigens or the at least one second antigens may include a purified antigen or be part of a cellular extract. The example kit may include one or more of the following features either alone or in combination.

The first solid support may include a first label and the second solid support may include a second label. The first label may be different from the second label. The first antigens may include markers for a first disease or condition. The first label may be associated with the first disease or condition. The at least one second antigen may include a marker for a second disease or condition. The second label may be associated with the second disease or condition.

The first antigens may include first labels that produce a signal in response to binding with corresponding antibodies. The at least one second antigen may include at least one second label that produces a signal in response to binding with at least one corresponding antibody. The first labels and the at least one second label may be of a same type or of a different type.

An example method includes contacting a biological sample from a subject with a complex that includes a solid support and at least two different types of antigens attached to the solid support; and detecting a presence of an antibody in the biological sample bound to the complex, where the presence of the antibody bound to the complex is indicative of a disease. The method may include one or more of the following features, either alone or in combination.

The method may include detecting the solid support based on a label on the solid support. The label may be associated the disease. Detecting may include detecting signals from labels on the at least two different types of antigens.

The complex may include one of multiple complexes that contacts the biological sample. Each of the multiple complexes may have a different configuration associated with a different disease. Each of the multiple complexes may include a respective solid support; and one or more antigens attached to the respective solid support.

At least part of the systems and techniques, including assays, described in this specification may be controlled by executing, on one or more processing devices, instructions that are stored on one or more non-transitory machine-readable storage media. Examples of non-transitory machine-readable storage media include read-only memory, an optical disk drive, memory disk drive, and random-access memory. At least part of the systems and techniques, including assays, described in this specification may be controlled using a computing system comprised of one or more processing devices and memory storing instructions that are executable by the one or more processing devices to perform various control operations. The compositions, devices, systems, and techniques, including assays, described in this specification may be configured, for example, through design, construction, formulation, arrangement, placement, programming, operation, activation, deactivation, and/or control.

Two or more of the features described in this specification, including in this summary section, may be combined to form implementations not specifically described in this specification.

The details of one or more implementations are set forth in the accompanying drawings and the description below. Other features and advantages will be apparent from the description and drawings, and from the claims.

### DESCRIPTION OF THE DRAWINGS

Figs. 1A, 1B, and 1C show a table of antigens and accession numbers therefor.
Fig. 2 is a drawing showing, conceptually, an example solid support-antigen complex containing a carrier and ten antigens.
Fig. 3 is a drawing showing, conceptually, an example solid support-antigen complex containing antigens attached to s carrier by linkers.
Fig. 4 is a drawing showing, conceptually, four example combinations of antigens on four different solid supports.
Fig. 5 is a drawing showing, conceptually, four example combinations of antigens on four different solid supports.
Fig. 6 is a drawing showing, conceptually, four example combinations of antigens on four different solid supports.
Fig. 7 shows photographs of three examples of autoantibodies that generate a positive indirect immunofluorescence result, but that are not routinely used in the differential diagnosis of connective tissue disease (CTD).
Fig. 8 is a flowchart showing example operations included in a process (assay) that uses a solid support-antigen complex to determine the likelihood that a subject has a disease, condition, or analyte.
Fig. 9 is a plot showing an example receiver operating characteristic (ROC) curve analyses depicting the ability to detect autoantibodies to individual autoantigens (Ro52, Ro60, RNP, La/SS-B) using a mixture of antigens coupled to a single bead.
Fig. 10 is a bar chart showing a comparison of the reactivity of individual antigens relative to the reactivity of individual antigens coupled to beads.
Fig. 11 is a plot showing results of a clinical study that screened biological samples for a disease using ten antigens on one solid support and using an FDA (Food and Drug Administration) cleared device.
Fig. 12 is a plot showing how results of a clinical study that screened for a disease using ten antigens compared to test results obtained using prior techniques and thousands of samples.
Fig. 13 is a plot showing results of a clinical study that screened for a disease using ten antigens on one solid support and how those results compared to test results obtained using prior techniques and thousands of samples.

Like reference numerals indicate like elements.

### DETAILED DESCRIPTION

Described herein are examples of multi-analyte assays for screening biological samples for antibodies, such as antinuclear antibodies (ANAs) / anti-cellular antibodies (ACA). In some implementations, the multi-analyte assays are configured to cover more analytes than ANA solid phase assays that screen for ANAs, and approach broader coverage of antibodies that the ANA HEp-2 cell assay detects.

An example antigen is a molecule, moiety, foreign particulate matter, or an allergen that can bind to a specific antibody. Antigens can be chemically synthesized, recombinantly synthesized, or isolated from a natural source.

An antibody includes a binding agent configured for specific binding to an antigen. An example antibody includes a polypeptide product of B-cells or recombinant equivalents thereof, that is able to bind to a specific molecular antigen and is composed of two heavy chains and two light chains. Each amino-terminal portion of each chain includes a variable region that confers binding specificity. Antibodies may include full length antibodies as well as functional fragments such as those antibodies. An example functional fragment, when used in reference to an antibody, incudes to a portion of the antibody including, e.g., heavy or light chain polypeptides that retain some or all of the binding activity as the antibody from which the fragment was derived. Such functional fragments can include, for example, an Fd, Fv, Fab, F(ab'), F(ab) 2, F(ab') 2, single chain Fv (scFv), diabody, triabody, tetrabody and minibody, which can be found described in, for example, Harlow and Lane, Antibodies: A Laboratory Manual, Cold Spring Harbor Laboratory, New York (1989); Myers (ed.), Molec. Biology and Biotechnology: A Comprehensive Desk Reference, New York: VCH Publisher, Inc.; Huston et al, Cell Biophysics, 22:189-224 (1993); Pluckthun and Skerra, Meth. Enzymol., 178:497-515 (1989); and in Day, E.D., Advanced Immunochemistry, Second Ed., Wiley-Liss, Inc., New York, NY (1990). Antibody functional fragments such as these can be used in the assays described herein.

An ANA is a type of autoantibody. An autoantibody includes an antibody produced by a subject's own immune system that is directed against one or more of the subject's own polypeptides or antigens. Autoantibodies can be produced by a subject's immune system when the immune system fails to distinguish in whole or in part, between self-tissue constituents and non-self-tissue constituents. An example autoantibody includes an immunoglobulin (Ig) directed against a constituent of tissue of a subject that produces the autoantibody.

An example anti-X antibody, when used in reference to an ANA, includes an ANA specific to antigen X or pan-specific to one or more antigen Xs. For example, an anti-centromere antibody would be an ANA specific or pan-specific to centromere. An anti-X antibody can include any antibody class (IgG, IgM, IgD, IgA and IgE) and subclass (IgGI, IgG2, IgG3, IgG4, IgAl and IgA2) or a functional fragment thereof. In some implementations, an anti-X antibody includes a human ANA to human antigen X.

The example assays described herein attach multiple antigens to a solid support as part of the testing protocol. For example, two or more (e.g., multiple) types of antigens may be immobilized on a single solid support. The antigens immobilized on the single solid support create a solid support-antigen complex capable of detecting two or more types of antibodies, such as two or more types of ANAs.

In some implementations, the solid supports may be pre-prepared with antigens coupled using one or more binding techniques. The binding techniques may include, but are not limited to, covalent binding via carboxy groups, covalent binding through click chemistry, biotin - streptavidin, adsorption, His capture, or tags such as those identified below. Covalent binding reagents can include any chemical or biological reagent that can be used to covalently immobilize an antigen directly on a surface of a solid support. Covalent binding reagents may include a carboxyl-to-amine reactive group such as carbodiimides, e.g., EDC (1-Ethyl-3-(3-Dimethylaminopropyl) Carbodiimide) or DCC (N,N'-dicyclohexylcarbodiimide), an amine reactive group such as N-hydroxysuccinimide (NHS) ester or imidoesters, a sulfhydryl-reactive crosslinker such as maleimides, haloacetyls or pyridyl disulfides, a carbonyl-reactive crosslinker group such as, hydrazides or alkoxyamines, a photoreactive crosslinker such as aryl azides or dizirines, or a chemoselective ligation group such as a Staudinger reaction pair. Click chemistry binding can include alkene and tetrazole photoclick reaction, alkene and azide [3+2] cycloaddition, alkene and tetrazine inverse-demand Diels-Alder, Copper(I)-catalyzed azide-alkyne cycloaddition (CuAAC), strain-promoted azide-alkyne cycloaddition (SPAAC), strain-promoted alkyne-nitrone cycloaddition (SPANC), and reactions of strained alkenes.

Combining two or more antigens on a single solid support using covalent binding can be challenging in some cases, since different antigens may compete with each other to bind to the same part of the solid support. In some cases, if one or more antigens is prevented from binding to the solid support, testing using those antigen(s) will not occur. Different ratios of antigens and different strategies for coupling the antigens to the solid supports may sometimes be used to address these issues. For example, antigens with lower binding affinity can be added at a higher concentration to increase the amount bound to the solid support as compared to antigens with a higher binding affinity. This control of the ratios of the antibodies can also be used to account for differences in binding affinities between the various antibodies and their corresponding antigens. For example, if a first antigen only weakly binds to its corresponding antibody, the bead may require a higher amount of the first antibody on the bead than an amount of a second antibody with a higher binding affinity to its corresponding antibody.

Non-covalent binding can employ any chemical or biological reagent that can be used to immobilize an antigen non-covalently on a surface of a solid support, such as affinity tags including biotin or capture reagents such as streptavidin or anti-tag antibodies, such as anti-His6 or anti-Myc antibodies.

In some implementations, the example assays use carriers to bind to the antigens. For example, the carriers bind to the solid support and the antigens bind to the carriers. Accordingly, in these examples, the antigens attach to the solid support indirectly. This configuration may reduce the likelihood that individual antigens will compete in an unpredictable manner with each other to bind to the solid support, for example, by providing a uniform binding/reactive surface for the antibodies. Furthermore, types and/or quantities of antigens for binding to the carriers can be chosen as described above for directing binding, so that the different antigens can be bound in controlled ratios to the carriers. In some implementations the ratios between a first and a second antigen can be in a range of about 10:1 to 1:10 by mole.

Examples of carriers that may be used include, but are not limited to, proteins such as streptavidin, bovine serum albumin (BSA), human serum albumin (HSA), and protein conjugates such as polymerized streptavidin. The carriers can include crosslinking agents such as glutaraldehyde, diisocyanates, carbodiimides ( e.g., 1-ethyl-3-[3-dimethyl aminopropyl] carbodiimide and N-hydroxysuccinimide), polyepoxy compounds (e.g., 1,4-butanediol diglycidyl ether and phenyl glycidyl ether), acyl azide, dithiobis succinimidyl propionate, disuccinimidyl suberate (DGS), ethylene glycol bis(succinimidyl succinate) (EGS), succinimidyl 4-(N-maleimidomethyl)cyclohexane-1-carboxy-(6-amidocaproate) (LC-SMCC), succinimidyl 4-(p-maleimidophenyl)butyrate (SMPB), formaldehyde, nitrogen mustards and adipic acid dihydrazide (ADH). Other carriers can include synthetic or naturally derived polymers such as dendrons and hyperbranched polymers. Examples of dendrons and hyperbranched polymers include Polyester-8-hydroxyl-1-acetylene bis-MPA dendron generation 3 (Sigma Aldrich@, product no. 686646), Polyester-16-hydroxyl-1-acetylene bis-MPA dendron, generation 4 (Sigma Aldrich@, product no. 686638), Polyester-32-hydroxyl-1-acetylene bis-MPA dendron, generation 5 (Sigma Aldrich@, product no. 686611), Polyester-8-hydroxyl-1-carboxyl bis-MPA dendron, generation 3 (Sigma Aldrich@, product No. 686670), Polyester-16-hydroxyl-1-carboxyl bis-MPA dendron, generation 4 (Sigma Aldrich@, product No. 686662), Polyester-32-hydroxyl-1-carboxyl bis-MPA dendron, generation 5 (Sigma Aldric^{®}, product no. 686654), Hyperbranched bis-MPA polyester-16-hydroxyl, generation 2 (Sigma Aldrich@, product no. 686603), Hyperbranched bis-MPA polyester-32-hydroxyl, generation 3 (Sigma Aldrich@, product no. 686581), and Hyperbranched bis-MPA polyester-64-hydroxyl, generation 4 (Sigma Aldrich@, product no. 686573). Examples of naturally derived polymers include polysaccharides such cellulose, chitin, chitosan, alginate, and hyaluronic acid, protein and polymer conjugates. In some implementations, carriers that may be used include, streptavidin, bovine serum albumin (BSA), human serum albumin (HSA), and protein conjugates such as polymerized streptavidin.

In some implementations, two or more different antigens may be bound to the same carrier, thereby increasing the number of antigens that may be attached (indirectly) to the solid support substrate. Different antigens may be bound to the carrier using linkers, such as biotin, polyethylene glycol (PEG), his-tags, SpyTag with SpyCatcher technology, or other small-molecule synthesized proteins or peptides.

Examples of antigens that may be attached, directly or indirectly via a carrier, to a single solid support at the same time include, but are not limited to, the following: Scl-70, centromere, dsDNA, Ro52, Ro60, SS-B, Ribo-P, DFS-70, EJ, Sp100, Gp210, actin, AMA-M2, AMA-M2/MIT3, TIF1-γ, NXP-2, SAE, PL-7, PL-12, OJ, HMGCR, SRP, Mi-2, MDA-5, RNA Helicase, Ki/SL, Sm/RNP, SS-A, PM/Scl, RNA Pol III, Th/To, Fibrillarin, Sm, Ku, Jo-1, RNP, BICD2, HK, KL, LC1, LKM-1, SLA, SS-A/Ro52, Scl-70, Ku, Centromere proteins, U11/U12 RNP, Hexokinase-1, and Kelch-12. In some implementations the antigens are native antigens, recombinant antigens, or smaller peptide sequences that target the specific antibody epitope of the whole protein.

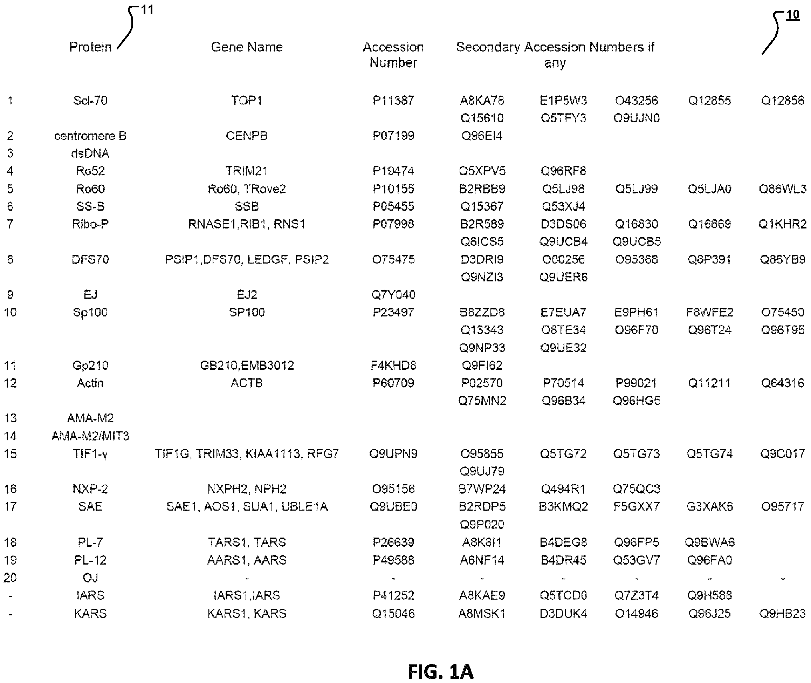

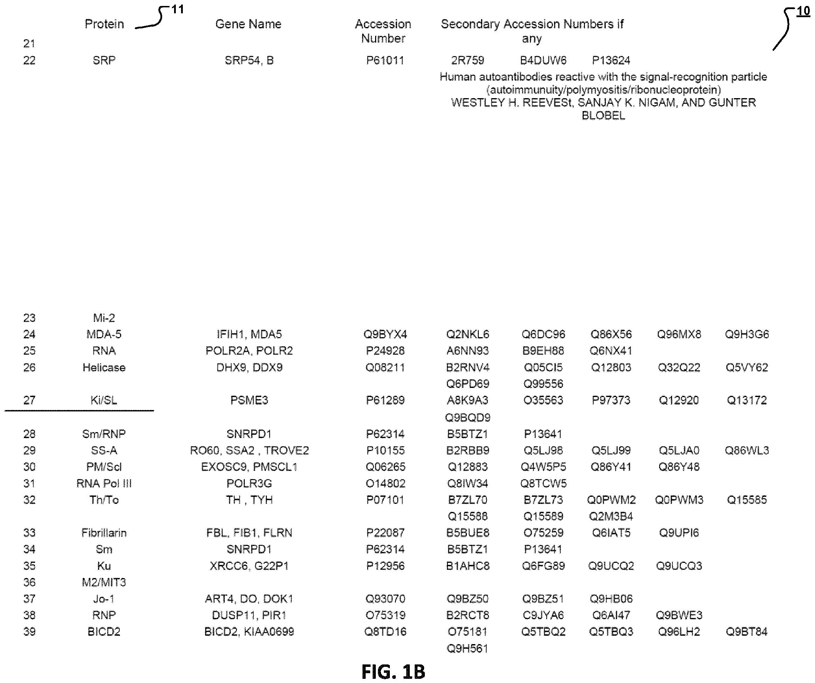

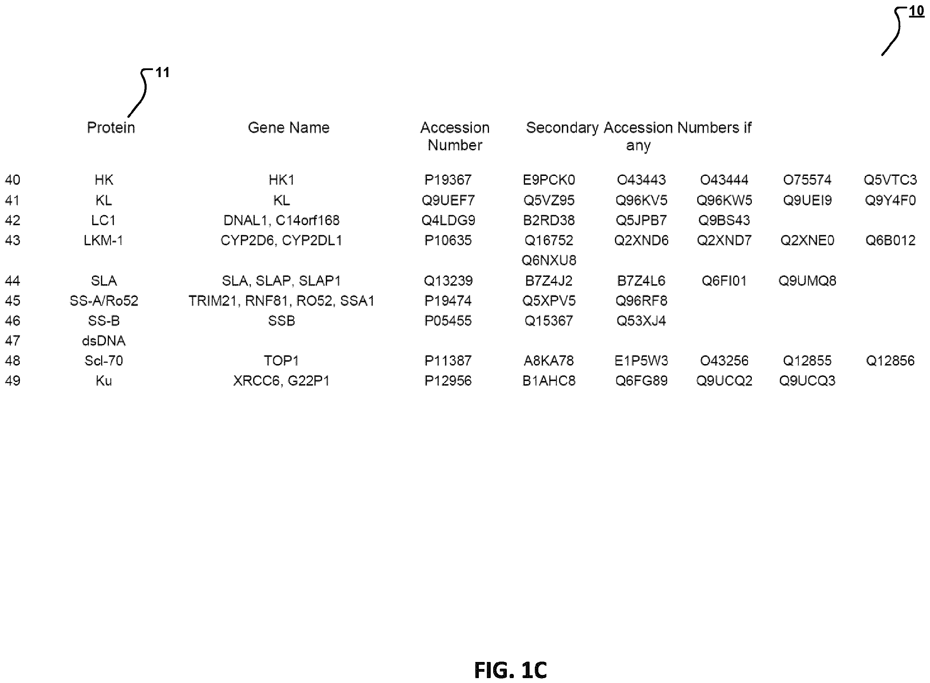

Table 10, shown across Fig. 1A, 1B, and 1C, shows examples of antigens 11 (proteins) that may be attached, directly or indirectly via s carrier, to a single solid support at the same time, and accession numbers therefor. Any combination of two or more of the antigens shown in Fig. 1A, 1B, and 1C and/or listed above may be attached to the same solid support at the same time. Any combination of two or more of the antigens shown in Fig. 1A, 1B, and 1C and/or those listed above may be attached to the same carrier (which is attached to solid support) at the same time.

In some implementations, the antigens may be parts of cell extracts or fragments. An example cell extract or fragment includes an intact part of a cell that has not been distilled or purified into individual antigens. An example cell extract may contain multiple antigens. Accordingly, the cell extract itself may attach to the solid support using any of the techniques described herein - for example, direct attachment or attachment to a carrier either directly or through a linker. Cell extracts may be attached to the same solid support as purified antigens or to a different solid support(s). Cell extracts attached to a solid support may be tested using the assays described herein in the same manner as purified antigens attached to the solid support.

In an example, a multi-analyte assay may include individual and purified antigens and cellular extracts (e.g. an HEp-2 cell extract), which are attached to different solid supports, but which are analyzed together to increase the performance of an ANA/ACA test. In an example, cellular extracts and purified antigens are used in combination, but on different solid supports. By binding the cellular extracts to a solid support, an assay may test for all of the antigens in the cellular extract.

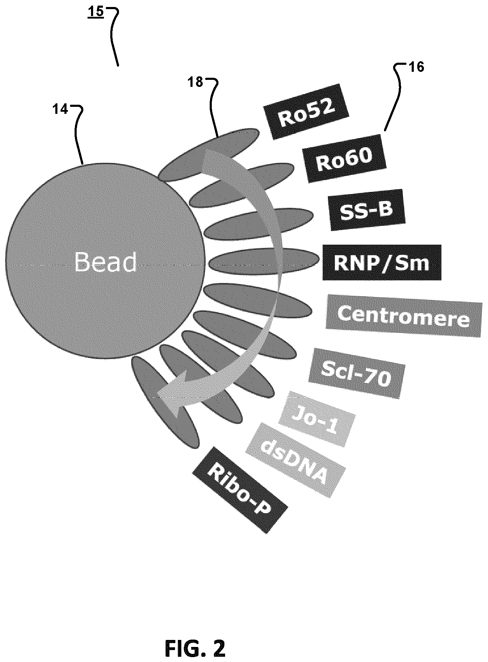

Fig. 2 shows an example of a solid support 14 - in this example, a bead - to which are attached ten antigens 16 via respective carriers 18. In this example, there is a single antigen attached to a single corresponding carrier, except for RNP/SM which is a complex of two antigens. The following table shows the relative molar ratio of antigens in the preparation to attach the antigens.

| Analyte | Ratio |
|---|---|
| Ro52 | 1.30 |
| Ro60 | 1.13 |
| SSB | 1.46 |
| RNP | 1.00 |
| Centromere | 1.17 |
| Scl-70 | 1.00 |
| dsDNA | 22.24 |
| Jo-1 | 1.62 |
| Ribo-p | 25.03 |

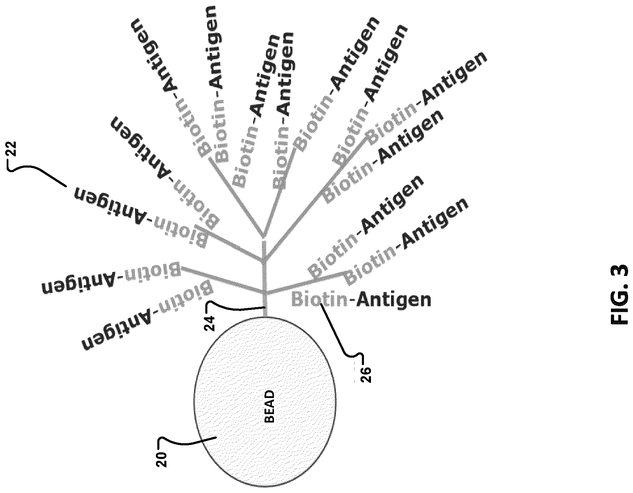

Fig. 3 shows an example of a solid support 20 - in this example, a bead - to which are attached fifteen antigens 22 via a respective linker and carrier. In this example, the fifteen antigens 22 are each attached to the same carrier 24 via a respective linker 26, which is biotin here. Although only one carrier 24 is attached to solid support 20 in Fig. 3, multiple carriers, each having multiple antigens attached thereto, may be attached to solid support 3.

Any number of antigens can be attached, directly or indirectly (e.g., through a carrier with or without a linker), to a single solid support. For example, two, three, four, five, six, seven, eight, nine, ten, eleven twelve, thirteen, fourteen, fifteen, sixteen, seventeen, eighteen, nineteen, twenty or more antigens can be attached, directly or indirectly, to a single solid support. In an example, two, three, four, five, six, seven, eight, nine, ten, eleven twelve, thirteen, fourteen, fifteen, sixteen, seventeen, eighteen, nineteen, twenty or more antigens may be attached to each carrier.

An example solid support, solid surface, or other grammatical equivalent includes any material that is appropriate for, or can be modified to be appropriate for, the attachment of antigens that can bind an autoantibody. Example materials include, for example, glass and modified or functionalized glass, plastics (including acrylics, polystyrene, methylstyrene, polyurethanes, Teflon^{™}, etc.), paramagnetic materials, thoria sol, carbon graphite, titanium oxide, latex or cross-linked dextrans such as sepharose, cellulose polysaccharides, nylon or nitrocellulose, ceramics, resins, silica or silica-based materials including silicon and modified silicon, carbon metals, inorganic glasses, optical fiber bundles, and a variety of other polymers.

In some implementations, the solid support may be or include a well or an array of wells or depressions in a surface. These can be fabricated using techniques such as photolithography, stamping, molding, and micro-etching. In some implementations, a solid support can include a multiwell plate such as a 96-, 384- or 1536-well plate. In some implementations, the solid support can be located within a flow cell or flow cell apparatus (e.g., a flow cell on a Biacore^{™} chip or a protein chip).

An example solid support may be or include a bead, sphere, particle, membrane, chip, slide, or test tube. Beads may include spheres or particles. Spheres, particles, or grammatical equivalents thereof may include discrete, non-planar particles, e.g., in the micrometer or nanometer dimensions. Spheres can include, for example, microspheres. Particles can include, for example, nanoparticles. In some implementations a bead can be irregular in shape. Alternatively or additionally, the beads can be porous. Example bead sizes range may from nanometers to millimeters, with beads from about 0.2 to about 200 microns being used in some implementations. In some implementations, bead size can range from about 0.5 to about 5 microns. In some implementations, beads smaller than 0.2 microns and larger than 200 microns can be used.

A solid support may be or include a patterned surface (e.g., on a protein chip) suitable for immobilization of purified proteins (e.g., an antigen) in an ordered pattern. An example patterned surface includes an arrangement of different regions in or on an exposed layer of a solid support. For example, one or more of the regions can be features where one or more purified proteins are present. The features can be separated by interstitial regions where purified proteins are not present. In some implementations, the pattern can be an x-y format of features that are in rows and columns. In some implementations, the pattern can be a repeating arrangement of features and/or interstitial regions. In some implementations, the pattern can be a random arrangement of features and/or interstitial regions. Examples of patterned surfaces that can be used in the example assays and complexes are described in U.S. Patent Application Publication No. 2008/0280785, U.S. Patent Application Publication No. 2003/0153013, and PCT Publication No. WO2009/039170, each of which is incorporated herein by reference.

The example solid supports described herein can also be used in the kits and techniques (e.g., the methods / assays) described herein.

Different types of antigens may be attached to the same solid support using the same or different immobilization techniques. In this regard, "immobilized" is used herein interchangeably with "attached", and both terms are intended to include, but are not limited to, covalent attachment or binding and non-covalent attachment or binding, unless indicated otherwise, either explicitly or by context.

The types of antigens on a single solid support and/or ratio of antigens on the single solid support may vary based on the antibodies to be detected. The types of antigen and the ratio may vary by disease state or by group of antigens. In some implementations, the antigens are combined on a single solid support by disease area, such as the SjS/MCTD spectrum, SSc/Myositis, SLE, and liver diseases. Also, the ratios of antigens can be tailored to analytes or a disease to be detected.

For example, a combination of antigens on a solid support may be associated with a particular disease or condition. In an example, the combination of antigens on a solid support 14 of Fig. 2 is associated with connective tissue disease. As such, the complex 15 of Fig. 2 may be used to bind to antibodies in a biological sample of a subject that are indicative of connective tissue diseases in the subject. In another example, the complexes described herein may use antigens related to autoimmune liver diseases since some antibodies in this group of disease generate a positive ANA. Antigens related to autoimmune liver diseases can be bound to the solid support, as some antibodies in this group of disease generate a positive ANA. Attaching these antigens can improve the differential diagnosis of systemic autoimmune conditions.

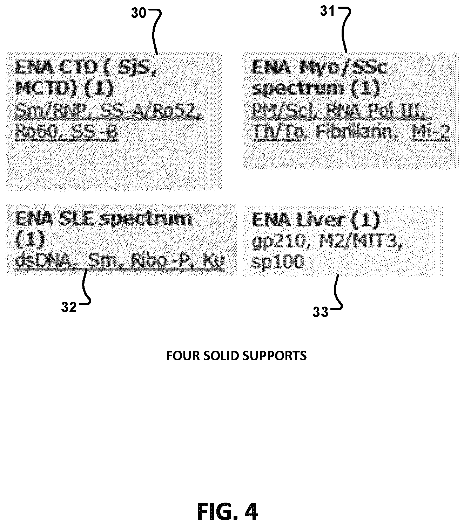

Groups of antigens and/or cell extracts attached to a single solid support may include antigens and/or cell extracts grouped by their disease area, such as, but not limited to:
1. One solid support (30, Fig. 4) for assaying for the SjS, MCTD spectrum including the antigens Sm/RNP, SS-A, Ro52, Ro60, and SS-B.
2. One solid support (31, Fig. 4) for assaying for SSc / Myositis including PM/Scl, RNA Pol III, Th/To, Fibrillarin, and Mi-2.
3. One solid support (32, Fig. 4) for assaying SLE including dsDNA, Sm, Ribo-P, and Ku.
4. One solid support (33, Fig. 4) assaying for liver autoimmune disease including gp210, AMA-M2/MIT3, and sp100.

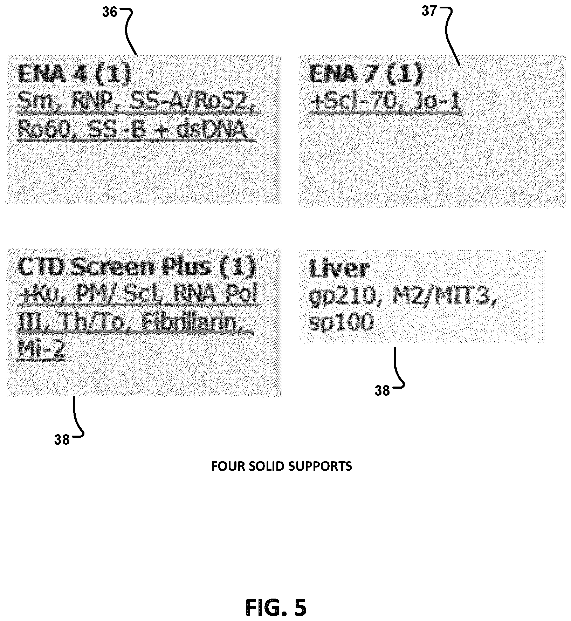

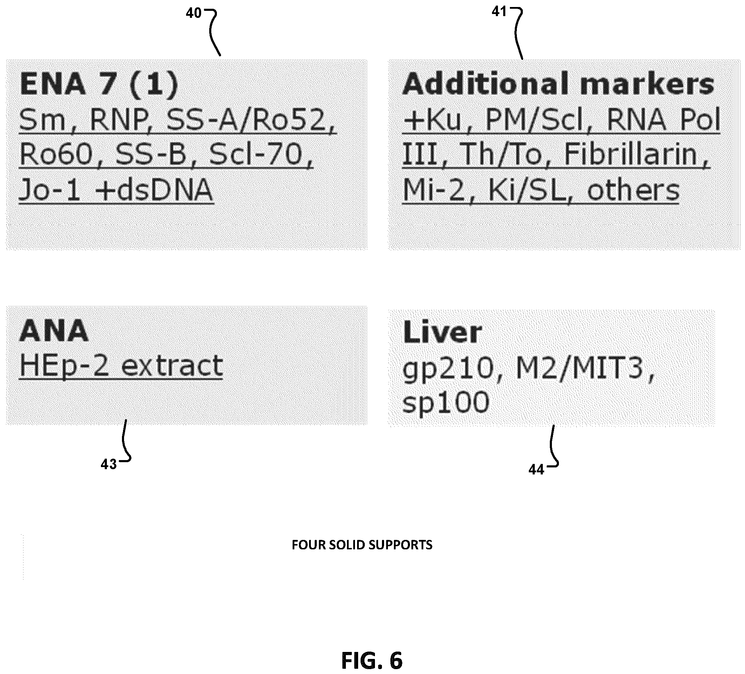

Groups of antigens and/or cell extracts attached to a single solid support may include antigens and/or cell extracts grouped by their importance/awareness and sensitivity, such as:
5. One solid support (ENA 4) (36, Fig. 5) including Sm, RNP, SS-A/Ro52, Ro60, and SS-B + dsDNA.
6. One solid support (ENA 7) (37, Fig. 5) including +Scl-70 and Jo-1.
7. One solid support (CTD Screen Plus) (38, Fig. 5) including +Ku, PM/Scl, RNA Pol III, Th/To, Fibrillarin, and Mi-2.
8. One solid support (Liver) (39, Fig. 5) including gp210, AMA-M2/MIT3, and sp100.
9. One solid support (40, Fig. 6) containing Sm, RNP, SS-A/Ro52, Ro60, SS-B, SCL-70, Jo-1 +dsDNA.
10. One solid support (41, Fig. 6) containing +Ku, PM/Scl, RNA Pol III, Th/To, Fibrillan, Mi-2, Ki/SL, and potentially one or more others.
11. One solid support (43, Fig. 6) containing an HEp-2 cell extract.
12. One solid support (44, Fig. 6) containing gp210, AMA-M2/MIT3, and sp100.
13. One solid support assaying for myositis specific antibodies including EJ, HMGCR, MDA5, Mi-2, NXP2, OJ, PL-7, PL-12, SAE, SRP, TIF1γ.
14. One solid support assaying for liver autoimmune disease including gp210, Hexokinase-1, Kelch-12, LC1, LKM-1, AMA-M2/MIT3, SLA, sp100.

In some implementations, combination of antigens and/or cell extracts may be used to detect analytes, rather than to determine whether a subject has a disease or condition. The analytes may be antibodies that bind to antigens on the solid support.

In some implementations, complexes used to detect different diseases or conditions may be combined in a single assay. For example, complexes used to detect different diseases may have different labels, such as chromatic labels, dyes, or others described herein. These labels make it possible to differentiate among the complexes in the same assay and therefore to identify which disease complex is registering positive for a particular disease. As a result, a single assay may be used to detect multiple diseases, which can save time and money. Analysis can also be simplified as compared to methods including multiple beads where multiple signals must be interpreted to determine the presence of the disease or condition. In some implementations, complexes used to detect analytes and complexes used to detect one or more diseases or conditions may be combined in a single assay.

Examples of diseases or conditions that may be detected or diagnosed using the assays and complexes described herein include, but are not limited to, systemic lupus erythematosus (SLE), Sjögren's syndrome (SjS), Systemic sclerosis (SSc), mixed connective tissue disease (MCTD), Idiopathic Inflammatory Myopathy (IIM), unknown/undifferentiated CTD, rheumatoid arthritis (RA), autoimmune hepatitis (AIH), antiphospholipid syndrome (APS), dense fine speckled or nucleolar IFA pattern, autoimmune gastritis (AIG), infectious HIV, HCV, Syphilis, Primary Biliary Cholangitis (PBC), ANCA associated vasculitis (AAV), and idiopathic inflammatory myopathies (IIM).

A label can be conjugated to any of the antigens described herein. Conjugation may include non-covalent or covalent cross-linkage. An example label may include a molecular entity that emits a signal and can be used as a readout or measurement for detection of an analyte. Various classes of labels exist. Examples of these classes include a fluorophore, an enzyme, a chemiluminescent moiety, a radioactive moiety, an organic dye, a small molecule, or a polypeptide or functional fragment thereof.

Examples of fluorophores include fluorescent dyes like phycoerytherin (PE), fluorescein isothiocyanate (FITC), tetramethylrhodamine (TRITC), BODIPY and AlexaFluor^{®} dyes. Fluorescent dyes can also include fluorescence resonance energy transfer (FRET)-dyes or time-resolved (TR)-FRET dyes. Fluorophore labels also include fluorescent proteins such as green fluorescent protein (GFP) and cyan fluorescent protein (CFP). Examples of enzyme labels include alkaline phosphatase (AP) or horseradish peroxidase (HRP). When any of the substrates S^'S^'-Tetramethylbenzidine (TMB), 3,3'-Diaminobenzidene (DAB) or 2,2'-azino-bis(3-ethylbenzothiazoline-6-sulphonic acid) (ABTS) are applied to HRP, a colored (chromogenic) or light (chemiluminescent) signal is produced. Examples of radioactive labels include, for example carbon-14 or Tritium, for example. Examples of small molecule labels include biotin, resins such as agarose beads and fluorescently labeled magnetic beads or nanoparticles such as colloidal gold. Examples of polypeptide or functional fragment labels include Avidin, Streptavidin or NeutrAvidin which have an affinity for biotin. Examples of polypeptide or functional fragment labels also include hemagglutinin (HA), glutathione-S-transferase (GST) or c-myc.

In some configurations, a label conjugated to an antigen includes an additional substrate or binding agent. As an example, an HRP label conjugated to an antigen includes a substrate to identify the antigen. Numerous other configurations for a label are known. Any labels may be used in the assays described herein. In some implementations, a label configuration can include PE conjugated to an antigen X, an antigen X:anti-X antibody complex binding agent, an anti-X antibody binding agent or an Ig binding agent. The labels described herein can be used in the assays described herein to detect the antibodies bound to the antigens. As used herein, "antigen X" includes but is not limited to, any antigen listed herein.

The ratios of antigens attached to a single solid support may be selected to achieve a desired sensitivity to one or more antibodies. For example, the ratio of antigens in a reaction mixture used to add the antigens to the solid support may be controlled to achieve a desired test sensitivity. This may include adding a greater amount of one type of antigen than one or more other types of antigens on a single solid support. For example, to detect lupus, additional amounts of dsDNA may be added to a reaction mixture for attachment to the solid support to boost signal for lupus. In some implementations, a bead containing cellular extracts (e.g., HEp-2 cells) can have a higher amount of the cellular extract by weight than an individual antigens and cellular extracts. For example, a cellular extract to the individual antigen can be greater than 1:1 by weight such as between 10:1 and 1:1.

The example assays described herein may use antigens to improve differential diagnosis of systemic autoimmune conditions. Examples of these assays may detect antibodies against cellular antigens by (i) combining different antigens on a single solid support with the same unique identifier in a multi-analyte assay, and/or (ii) providing antigens on a single solid support that are bound by autoantibodies directed to cellular antigens, but that are not typically for the diagnosis of CTD. Examples of such antigens include, but are not limited to, the antigens listed below in Table 1 below.

**Table 1: Intracellular autoantigens not associated with connective tissue disease, but targeted by antibodies that generate a positive ANA test when using HEp-2 IIF method**

| Antigen | Disease association |
|---|---|
| AMA-M2 / MIT3 | PBC (primary biliary cholangitis) |
| Sp100 | PBC |
| Gp210 | PBC |
| Actin | Autoimmune hepatitis |

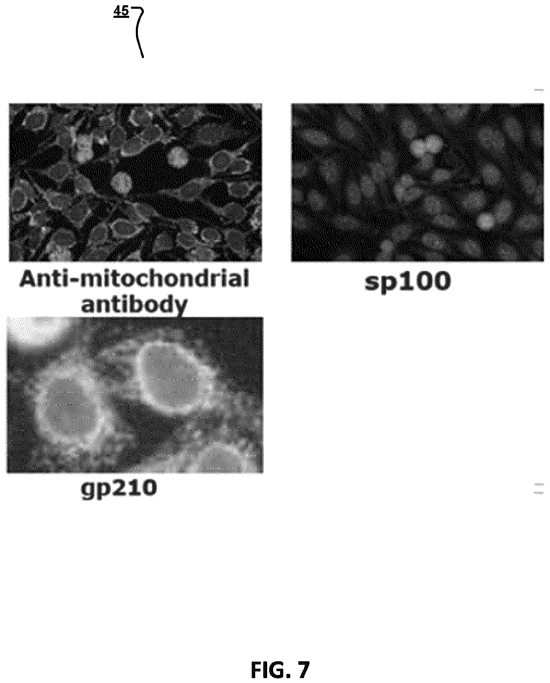

Fig. 7 also shows examples of autoantibodies 45 that generate a positive indirect immunofluorescence but that are not typically for the diagnosis of CTD.

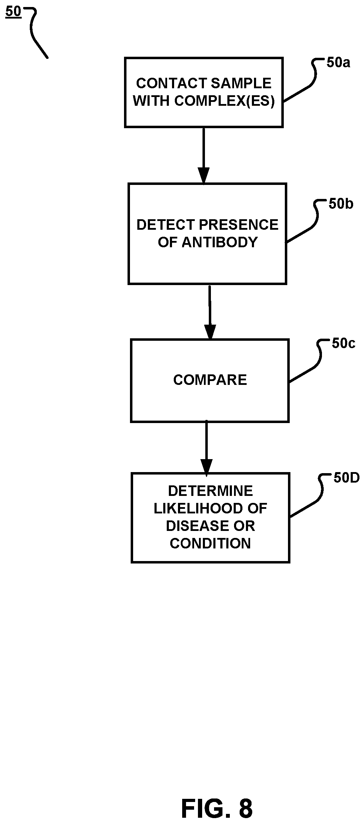

Fig. 8 is a flowchart showing operations included in an example process (assay) 50 for detecting or diagnosing a disease using solid support-antigen complexes of the type described herein. The operations include contacting (50a) a biological sample from a subject with one or more complexes of the type described herein. This may be performed by contacting the biological sample with a fluid containing the complexes Each complex may include a solid support and at least two different types of antigens attached to the solid support. Different ones of the complexes may be configured to detect antibodies associated with a different disease, condition, or analyte. The operations include detecting (50b) a presence of one or more antibodies in the biological sample that are bound to a complex.

The presence of the one or more antibodies bound to the complex is indicative of a disease associated with a complex. Detection may be performed using a diagnostic test instrument or detector to identify signals, such as fluorescence or chemiluminescence, from a complex and to identify a label associated with the complex emitting the signals. The levels (e.g., intensities) of the signals may be compared (50c) against predefined benchmarks to determine (50d) the likelihood that the subject has a disease condition associated with the antibodies bound to a complex.

By way of example, signals, such as fluorescent or chemiluminescent signals, for subjects known to have disease associated with each complex may be previously measured and used to create a curve that relates signal levels to likelihood that the subject has a particular disease. This curve may be retrieved in operation 50c. The levels of the signals measured may be compared to the curve to determine a likelihood that the subject has the disease associated with each complex. These operations may be performed for complexes associated with different diseases and for complexes associated with specific analytes. In the case of analytes, curves may have been generated beforehand relating signal levels to the presence of different analytes in test samples, which curves may be used to determine the likelihood of the presence of an analyte in the biological sample.

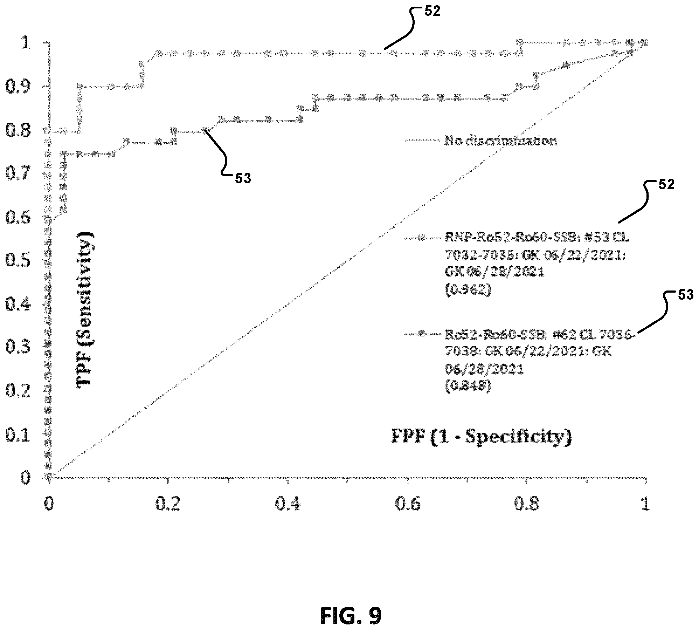

Fig. 9 shows a receiver operating characteristic (ROC) curve analysis discriminating between reactivity to antigens using a mixture of the four antigens shown, namely RNP, Ro52, Ro60, and SSB. Two formats are compared, where in a first format the antigens are all coupled to a single bead and in a second format each antigen is coupled to a different bead as a homogeneous population. Two different formulations 52 and 53 are shown each comparing the two formats. Sensitivity of the two formulations is plotted on the Y axis relative to specificity on the X axis. The plot shows that the specificity and sensitivity of the two formats are comparable.

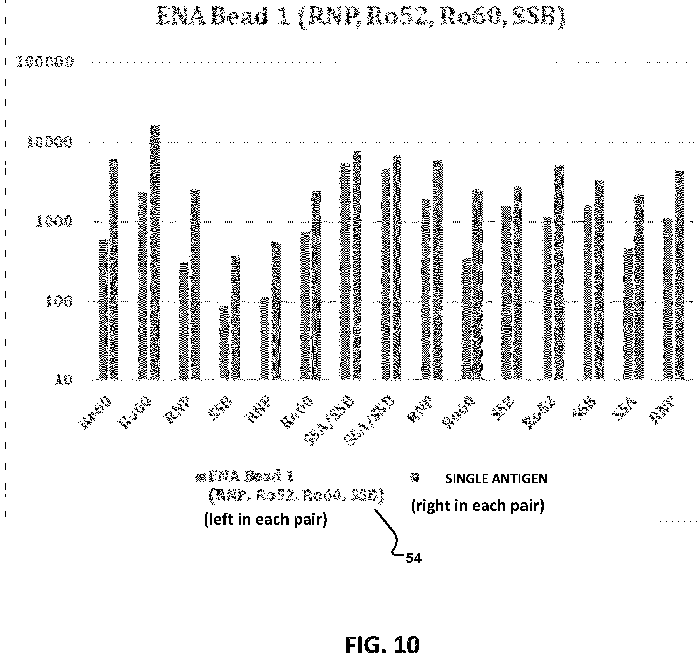

Fig. 10 is a bar chart showing a comparison of the reactivity of individual antigens relative the reactivity/signal of that same antigen included on a solid support (bead) containing RNP, Ro52, Ro60, and SSB. This illustrates that the reactivities for antigens coupled to a single support are within the same order of magnitude as those where only one antigen is coupled to a bead. It is noted that the reactivity/signal can be enhanced by including more surface density of the antibodies.

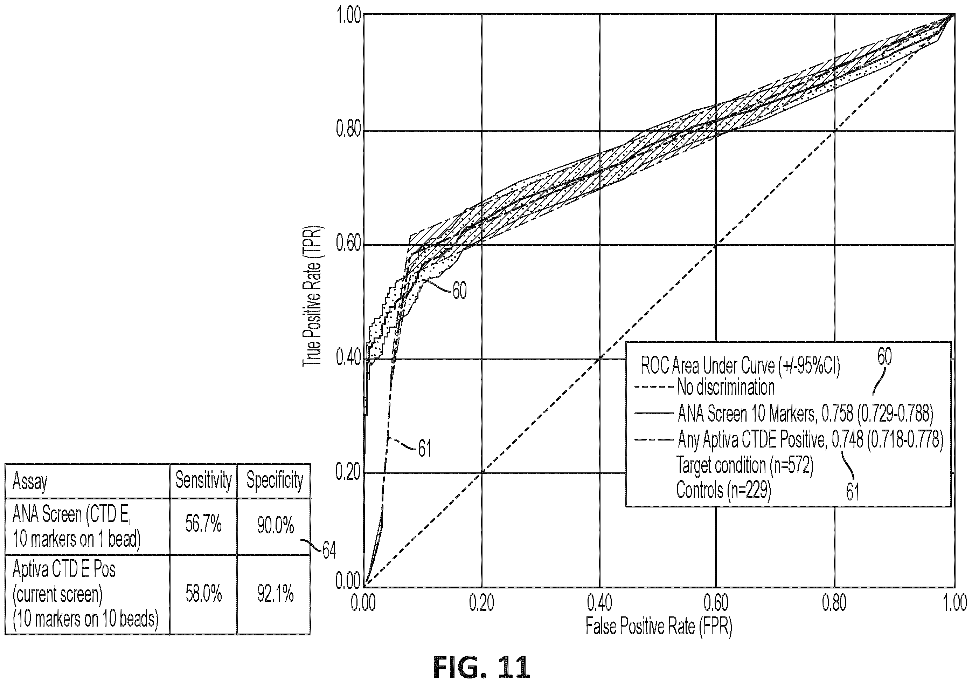

Fig. 11 is a plot showing results of a clinical study that screened biological samples for a disease using ten antigens on one solid support and using an FDA (Food and Drug Administration) cleared device, here the Aptiva^{®} system, which is a digital multi-analyte test system by Inova Diagnostics^{®}, Inc. The plot relates false positive rate to true positive rate for both techniques. Curve 60 produced using ten antigens on a solid support is similar curve 61 produced using the FDA cleared device. As shown in text box 64, the two assays have similar sensitivities and specificities, where sensitivity indicates the percentage of subjects accurately determined to have a disease and specificity indicates the percentage of subjects identified with the disease.

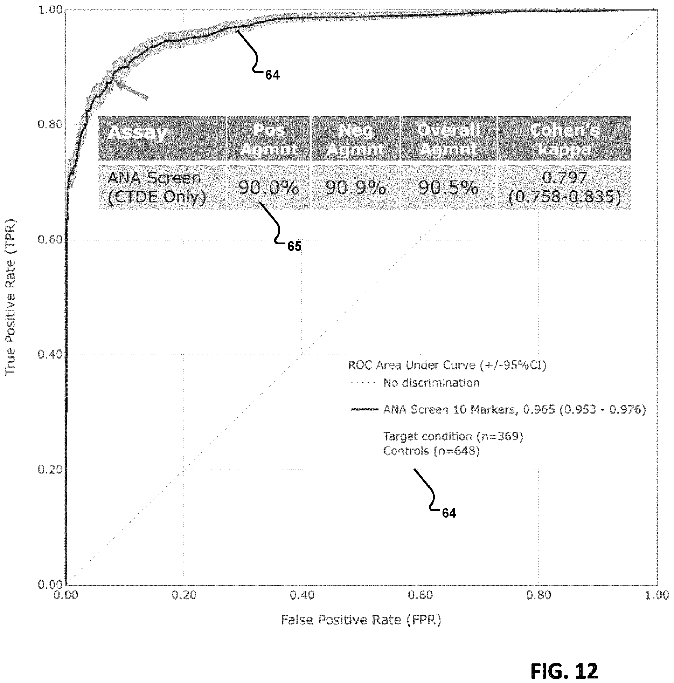

Fig. 12 is a plot showing how results of a clinical study that screened for a disease using ten antigens compared to test results obtained using prior techniques and thousands of samples. The prior techniques include assays performed on the Quanta Flash^{®} system by Werfen^{®} S.A. The plot relates false positive rate to true positive rate. Curve 64 shows that results produced using ten antigens on one solid support and those produced using prior techniques are over 90% in agreement. Positive, negative, and overall agreement values are shown in text box 65.

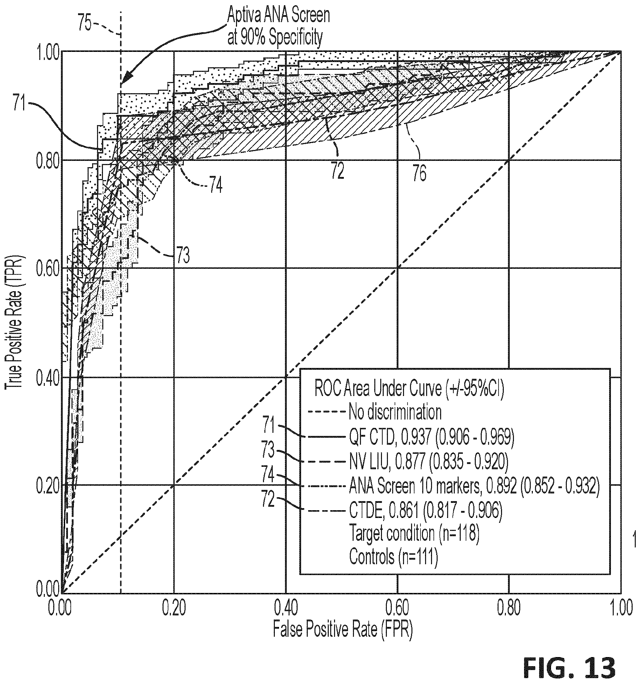

Fig. 13 is a plot showing results of a clinical study that screened for a disease using ten antigens on one solid support and how those results compared to test results obtained using prior techniques and thousands of samples. The plot relates false positive rates to true positive rates. The prior techniques include assays performed on the Quanta Flash^{®} system, the Nova View^{®} digital IFA reader, and the Aptiva^{®} system. Curve 71 is from the Quanta Flash^{®} system, curve 72 is from the Aptiva^{®} system; curve 73 is from the Nova View^{®} reader, and curve 74 is from the assay described herein using ten antigens on one solid support. As shown in the figure, the curve 74 is from the assay described herein using ten antigens on one solid support has a sensitivity 75 of close to 90%, which is commensurate with that of the Aptiva^{®} system. Note that the shading (e.g., 76) associated with each curve corresponds to a 95% confidence level in the data. The overlap in shading among the curves is an indicator that the assay described herein using ten antigens on one solid support has an accuracy that is commensurate with those of the prior techniques.

The example assays described herein may be implemented in a cartridge and/or a kit that operates with a multi-analyte test system such as the Aptiva^{®} system or the systems noted above. In an example implementation, two or more solid supports (for example, two, three, four, five, six, and so forth), each having attached a combination of two or more antigens either directly or indirectly through the use of carrier(s) or both carrier(s) and linker(s), are mixed together and then filled in tubes which may be part of the cartridge. The remaining reaction follows the Aptiva^{®} protocol.

In an example protocol, antigens are attached to paramagnetic particles that carry unique labels or signatures and that are incubated with diluted patient samples, which may be a bodily fluid such as blood or saliva. After 9.5 minutes of incubation at 37°C, particles are washed and incubated for 9.5 minutes more. The particles are then incubated at 37°C with anti-human IgG conjugated to phycoerythrin (PE) to label bound autoantibodies. After a final wash cycle, median fluorescence intensity (MFI) on the particles is captured using a digital imager and the captured test result is analyzed using processes to extract meaningful information for each analyte. Detection may also be performed using indirect immunofluorescence assay (IFA).

In cartridge-based systems that include multiple antigens attached to a single solid support, it is possible to use fewer solid supports than in techniques that use one antigen per single solid support. This may result in a reduced number of solid supports, which can reduce the number of cartridges needed to achieve the same number of tests. Yet, the multiple types of antigens can capture increased types of target antibodies and allow for comprehensive analyses of the test sample. In addition, by selecting the types and ratios of antigen per solid support, test sensitivity can be improved relative to techniques that use one antigen per solid support.

In some implementations, a single cartridge such as those described herein may include one or more (e.g., four) solid supports containing groups of antigens 1 to 4 above. In some implementations, a single cartridge such as those described herein may include one or more (e.g., four) solid supports containing groups of antigens 5 to 8 above. In some implementations, a single cartridge such as those described herein may include two or more (e.g., four) solid supports containing groups of antigens 5 to 12 above.

Currently, the most common technique to detect ANA as a part of the diagnosis of ANA-associated rheumatic disease is the HEp-2 IIF test, which is known for high sensitivity, but lower specificity. Solid phase bead-based systems may have lower sensitivity than such systems. Based on previous studies, the sensitivity of such solid phase assays is about 75% depending on the composition of the cohort. In this context, a sensitivity of 75% means that an assay to detect a disease identifies, among a group of test subjects known to have the disease, 75% of the test subjects as having the disease. Including multiple antigens per solid support has the potential to increase the sensitivity of the assay by about 10%. Including multiple antigens per solid support may also increase assay performance, especially in cases systemic sclerosis and myositis.

Consequently, the example assays described herein may expand the capabilities of multi-analyte test system like Aptiva^{®} such that those systems can provide test results that are on par with IIF on HEp-2 cells for the detection ANA.

Software that processes test results in a diagnostic test, such as those described herein, system and/or process 50 above may be configured to combine multiple results (from multiple antigens) to provide new indications or more accurate indications. Machine learning algorithms may be implemented by such software to provide these new indications or more accurate indications.

Also described herein are example implementations of kits for detecting, diagnosing and/or monitoring a disease or condition. An example kit can include one or more of the example solid support-antigen complexes described herein. For example, a kit can include a solid support-antigen complex used to detect a first disease, a solid support-antigen complex used to detect a second disease that is different from the first disease, a solid support-antigen complex used to detect a third disease that is different from the first and second diseases, and so forth. For example, a kit can include a solid support-antigen complexes used to detect multiple different analytes. A kit can include both solid support-antigen complexes to detect one or more different diseases and solid support-antigen complexes to detect one or more different analytes.

Examples of the kits described herein may include a detection probe specific to an anti-X antibody. An example detection probe can include antigens such as those described herein or antibody-specific binding polypeptides. Thus, in some examples, a detection probe specific for anti-X antibody may include, for example, an antigen X fragment, an antigen X:anti-X antibody complex binding agent, an anti-X antibody binding agent, or an Ig binding agent or protein. Example antibody specific binding polypeptides include any polypeptide that can specifically recognized antibodies as well as functional fragments thereof. Examples of antibody specific binding polypeptides include IgG binding proteins, receptors, chimeric receptors and binding polypeptides identified from screening of random or combinatorial libraries.

Examples of IgG binding proteins include protein A and protein G. Antibody specific binding polypeptides may be chemically synthesized or purified from a natural source or recombinantly made. The antibody specific binding polypeptides described herein can be mammalian, including mouse, rabbit, goat, chicken, donkey and the like.

When referring to binding to a target such as an antibody, a detection probe can bind a target directly or it can bind to the target indirectly. A direct binder includes, for example, an antibody or other antibody specific binding polypeptide that specifically recognizes an antigen X:anti-X antibody complex as well as an antibody or other antibody specific binding polypeptide that specifically binds to an anti-X antibody. A detection probe can be made specific to the target using anti-Ig or other antibody specific binding polypeptide that binds Ig. Such antibodies and antibody specific binding polypeptides can be made specific to an anti-X antibody by, for example, capturing the anti-X antibody with an antigen X herein and washing away non-anti-X Ig prior to adding anti-Ig or other antibody specific binding polypeptide that binds Ig. Other configurations for isolating or separating such a binding complex in order to achieve specific binding to a target can be used as an indirect mechanism to make a detection probe specific to a target. Thus, a "detection probe specific to an anti-X antibody" may include, for example, an antigen X, an antigen X:anti-X antibody complex binding agent, an anti-X antibody binding agent, and an Ig binding agent.

The detection probe can include a label or reporter tag. An example label or reporter tag includes one or more molecules configured to produce a signal indicative of detection of an anti-X antibody to which the detection probe binds. Labels or reporter tags can be attached or conjugated, for example, to the detection probe through non-covalent or covalent cross-linkage. Non-covalent and covalent immobilization of reporter tags to detection probes can be performed by techniques as described in, for example, Dennler et al, "Antibody conjugates: from heterogeneous populations to defined reagents," Antibodies, 4:197-224 (2015). Labels or reporter tags produce various signals, depending on the type of reporter tag.

An example kit can include a solid support, examples of which are described herein. The kit can also include a control. An example control includes a positive or negative standard for comparison against a tested sample where the existence or amount of anti-X antibody is unknown. A kit can include a positive control. In some implementations, a positive control includes a sample containing a detectable amount of anti-X antibody or functional fragment thereof or levels above the threshold. In some implementations, a positive control can be obtained from a diseased subject who has levels of anti-X antibody above threshold. Additionally or alternatively, a positive control can contain an antibody or functional fragment thereof specific to an antigen. The antibody or functional fragment thereof can be selected from a monoclonal or polyclonal antibody. In other implementations, the kit can include a negative control. A negative control can include a sample containing no detectable amount of anti-X antibody or functional fragment thereof or levels below the threshold. In some implementations, a negative control can be obtained from a healthy control individual or can be synthesized in vitro. For example, a negative control can include water or buffer.

An example kit can include a carrier, examples of which are described herein. The example kit can include a linker, examples of which are described herein.

An example kit can include one or more ancillary reagents. An example ancillary reagent includes a substance, mixture, material or component that is useful to implement an assay or composition. Example ancillary reagents include a conjugation reagent, a buffer, instructions, instruments and the like.

In some implementations, a kit can include a reagent and the reagent used in the kit can include any conjugation reagent known in the art, including covalent and non-covalent conjugation reagents. Covalent conjugation reagents can include any chemical or biological reagent that can be used to covalently immobilize a polypeptide on a surface. Covalent conjugation reagents can include a carboxyl-to-amine reactive group such as carbodiimides such as EDC or DCC, an amine reactive group such as N-hydroxysuccinimide (NHS) ester or imidoesters, a sulfhydryl-reactive crosslinker such as maleimides, haloacetyls or pyridyl disulfides, a carbonyl- reactive crosslinker groups such as, hydrazides or alkoxyamines, a photoreactive crosslinker such as aryl azides or dizirines or a chemoselective ligation group such as a Staudinger reaction pair. Non-covalent immobilization reagents can include any chemical or biological reagent that can be used to immobilize a polypeptide non-covalently on a surface, such as affinity tags such as biotin or capture reagents such as streptavidin or anti-tag antibodies, such as anti-His6 or anti-Myc antibodies.

An example reagent can include combinations of conjugation reagents. Such combinations include, e.g., EDC and NHS, which can be used, e.g., to immobilize a protein on a surface, such as a carboxylated dextrane matrix (e.g., on a BIAcore^{™} CM5 chip or a dextrane-based bead). Combinations of conjugation reagents can be stored as premixed reagent combinations or with one or more conjugation reagents of the combination being stored separately from other conjugation reagents.

In some implementations, a reagent used in a kit can include a coating buffer. A coating buffer can include sodium carbonate-sodium hydroxide or phosphate. In some implementations, the coating buffer can be 0.1M NaHCCb (e.g., about pH 9.6).

In some implementations, the reagent can include a wash buffer. Awash buffer can include tris (hydroxymethyl)aminomethane (Tris)-based buffers like Tris-buffered saline (TBS) or phosphate buffers like phosphate-buffered saline (PBS). Wash buffers can be composed of detergents, such as ionic or non-ionic detergents. In some implementations, the wash buffer can be a PBS buffer at about pH 7.4 including Tween^{®}20 at about 0.05%.

In some implementations, the reagent can include a dilution buffer. Any dilution buffer known in the art can be included in the kit. Examples of dilution buffers include a protein such as bovine serum albumin (BSA) and a detergent such as Tween^{®}20. In some implementations, the dilution buffer can be PBS at about pH 7.4 including BSA at about 1% BSA and Tween ^{®}20 at about 0.05%.

In some implementations, the reagent can include a detection or assay buffer. Any detection or assay buffer known in the art can be included in the kit.

The detection or assay buffer can be a colorimetric detection or assay buffer, a fluorescent detection or assay buffer or a chemiluminescent detection or assay buffer. Colorimetric detection or assay buffers include, but are not limited to, PNPP (p-nitrophenyl phosphate), ABTS (2,2'-azino-bis(3- ethylbenzothiazoline-6-sulphonic acid)), or OPD (o-phenylenediamine). Fluorescent detection or assay buffers include QuantaBlu^{™} or QuantaRed^{™} (Thermo Scientific^{®}, Waltham, MA). Chemiluminescent detection or assay buffers can include luminol or luciferin. Detection or assay buffers can also include a trigger such as H₂O₂ and a tracer such as isoluminol-conjugate.

In some implementations, the reagent can include solutions useful for calibration or testing. In some implementations, the reagent can include a stop solution. Any stop solution known in the art can be included in the kit. Example stop solutions terminate or delay the further development of the detection reagent and corresponding assay signals. Stop solutions can include, e.g., low-pH buffers (e.g., glycine-buffer, pH 2.0), chaotrophic agents (e.g., guanidinium chloride, sodium-dodecyl sulfate (SDS)), or reducing agents (e.g., dithiothreitol, b-mecaptoethanol) or the like.

In some implementations, the reagent can include cleaning reagent(s). Cleaning reagents can include any cleaning reagent known in the art. In some implementations, the cleaning reagents can be the cleaning reagents recommended by the manufacturers of the automated assay systems.

In some implementations, a kit of the type described herein can be used to perform an assay on a test instrument such as the test instruments or systems described herein. In some implementations, detection instruments are configured to detect and to measure a label. To this end, the detection instruments may be configured for detecting or measuring fluorescence, luminescence, chemiluminescence or absorbance, reflectance, transmittance, birefringence or refractive index. In some implementations, detection instruments can implement confocal and non-confocal microscopy, a microplate reader, a flow cytometer and the like.

In some implementations, the kit can include a component suitable for collecting a biological sample as described below. A component can include collection tubes, columns, syringes, needles and the like.

In some implementations, the kit can include instructions for using the components of the kit. Instructions can be in any form, inside or outside of the kit. The instructions provide details regarding protocol and analytical techniques.

Components of a kit can be in varying physical states. For example, some or all of the components can be lyophilized or in aqueous solution or frozen. Such components may include two or more antigens to be attached to a solid support as described herein, the solid support, a detection probe, and ancillary reagents.

A kit of can be tailored to specific assay technologies. For example, in some implementations, the kits can be a FIA kit, a CIA kit, a RIA kit, a multiplex immunoassay kit, a protein/peptide array immunoassay kit, a SPRIA kit, an IIF kit, an ELISA, a particle based multi-analyte technology (PMAT kit) or a Dot Blot kit. In some implementations, the ELISA kits can include a washing buffer, a sample diluent, a secondary antibody-enzyme conjugate, a detection reagent and a stop solution. In some implementations, the Dot Blot kits can include a washing buffer, a sample diluent, a secondary antibody-enzyme conjugate, a detection reagent and a stop solution. In some implementations, the CIA kit can include a washing buffer, a sample diluent, a tracer (e.g., isoluminol-conjugate) and a trigger (e.g., H₂O₂). In some implementations, the multiplex kit can include a washing buffer, a sample diluent and a secondary antibody-enzyme conjugate. In some implementations, the kits can be tailored to the Luminex platform and include, as an example, xMAP^{®} beads.

A kit can be used to diagnose diseases such as, but not limited to, CTD or autoimmune diseases, by providing a mechanism for detecting anti-X antibody bound to an antigen X. A kit can be used to detect anti-X antibody using any of the techniques disclosed herein. The antigen X:anti-X antibody complex or antigenic fragment thereof can have a stoichiometry of one to one or more than one to one anti-X antibody. In some implementations, the complexes can have one anti-X antibody per antigen X. In some implementations, the complexes can have two anti-X antibodies per antigen X. In some implementations, the complexes can have more than two anti-X antibodies per antigen X. Techniques for measuring binding stoichiometries of two antigens are well known in the art and include, e.g., isothermal titration calorimetry (ITC) and ultracentrifugation.

In some implementations, the antigen X:anti-X antibody complex or antigenic fragment thereof, can include a plurality of complexes with identical stoichiometry. For example, all complexes in the plurality of complexes have one anti-X antibody per antigen X. In some implementations, the antigen X:anti-X antibody complex or antigenic fragment thereof, can be a plurality of complexes with different stoichiometries. For example, some complexes in the plurality of complexes can have one anti-X antibody per antigen X and some other complexes in the plurality of complexes can have more than one anti-X antibody per antigen X.

In some implementations, an antigen X can bind with an anti-X antibody with high affinity. In some implementations, anti-X antibody binding sites can be bound by anti-X antibody with more than 2-fold, more than 3 -fold, more than 4-fold, more than 5-fold, more than 8-fold, more than 10-fold, more than 15-fold, more than 20-fold, more than 25-fold, more than 50-fold, more than 100-fold, more than 300-fold, more than 1,000-fold, more than 3,000-fold, more than 10,000-fold, more than 30,000-fold or more than 100,000- fold greater binding affinity.

Greater binding affinities are evidenced by lower dissociation constants (KDS) for the antigen X:anti-X antibody complex or by higher association constants (KAS) for the respective anti-X antibody and antigen X. In some implementations, the dissociation constants for (KDS) for the X:anti-X antibody complex can be less than 1 mM (millimolar), less than 300 nM (nanomolar), less than 100 nM, less than 30 nM, less than 10 nM, less than 3 nM, less than 1 nM, less than 300 pM, less than 100 pM, less than 30 pM, less than 10 pM, less than 3 pM or less than 1 pM. Techniques for measuring binding affinities of antibodies to antigens are known and include ELISA, isothermal titration calorimetry (ITC) and surface plasmon resonance (SPR)

The presence of increased anti-X antibody in a subject compared to a healthy control individual can be indicative of the presence of a disease or the risk of developing a disease. Accordingly, a measurable increase in an anti-X antibody to antigen X is used to diagnose a disease associated with an anti-X antibody. Examples of techniques for detection and comparison of anti-X antibody levels to a control (e.g., a predefined baseline) are provided below.

In some implementations, the level of anti-X antibody is detected. In some implementations, antigen X:anti-X antibody complexes can be formed using the compositions and techniques described herein and an anti-X antibody in the complex can be detected.

In some implementations, detection of an increased level of anti-X antibody compared to a healthy control individual is indicative of a subject having a disease associated with an anti-X antibody. In some implementations, following diagnosis of a disease associated with an anti-X antibody using the support-antigen complexes and techniques described herein, the presence of a disease associated with an anti-X antibody can be further corroborated based on a variety of symptoms associated with the onset or presence of a disease associated with an anti-X antibody. For example, clinical symptoms associated with systemic sclerosis and myositis.

In some implementations, detection of an increased level of anti-X antibody compared to a healthy control individual indicates that the subject is at risk of developing clinical symptoms of a disease associated with anti-X antibody. In some implementations, a subject can be at risk of developing clinical symptoms of a disease associated with anti-X antibody within less than 3 months, less than 6 months, less than 9 months, less than 12 months, less than 18 months, less than 2 years, less than 3 years, less than 4 years, less than 5 years, less than 6 years, less than 7 years, less than 8 years, less than 9 years, less than 10 years, less than 12 years, less than 14 years, or less than 16 years from the determination of the increased anti-X antibody level.

In some implementations, the presence of an increased level of anti-X antibody compared to a healthy control individual indicates that the subject is more than 5%, more than 10%, more than 15%, more than 20%, more than 25%, more than 30%, more than 35%, more than 40%, more than 45%, more than 50%, more than 60%, more than 70% or more than 80%, or more than 90% likely to develop clinical symptoms of a disease associated with anti-X antibody within 5 years following the determination of increased anti-X antibody. In some implementations, the presence of an increased level of anti-X antibody can indicate that the subject is more than 2-fold, more than 3-fold, more than 4-fold, more than 5-fold, more than 6-fold, more than 7-fold, more than 8-fold, more than 9-fold, or more than 10-fold likely to develop clinical symptoms of a disease associated with anti-X antibody within 5 years following determination of increased anti-X antibody level compared to a healthy control individual.

The terms "subject," "organism," "individual" or "patient" are used as synonyms and interchangeably and may refer to a vertebrate mammal. As an example, the subjects of this disclosure can include healthy subjects, asymptomatic subjects and diseased subjects.

A subject can be "suspected of having a disease associated with anti-X antibody" as determined by the presence of certain risk factors that are known in the art. Example risk factors include, for example, a genetic predisposition, a personal disease history, a lifestyle factor, an environmental factor, a diagnostic indicator and the like.

A subject at risk for having or developing a disease associated with anti-X antibody can have a genetic predisposition for developing a disease associated with anti-X antibody or a family history of a disease associated with anti-X antibody or other autoimmune diseases. The subject can be exposed to certain lifestyle factors (e.g., smoking cigarettes) or environmental factors promoting the development of a disease associated with anti-X antibody. The subject can show clinical disease manifestations of a disease associated with anti-X antibody. The subject can be a patient who is receiving a clinical workup to diagnose a disease associated with anti-X antibody or to assess the risk of developing a disease associated with anti-X antibody.

In some implementations, the subject can have anti-X antibodies in their bodily fluid (e.g., blood) or tissue. In some implementations, the subject can have elevated anti-X antibodies in their bodily fluid or tissue compared to normal healthy subjects. In some implementations, the subject will not have elevated anti-X antibodies in their bodily fluid or tissue compared to normal healthy subjects.

In some implementations, the subject can be treatment naive. In some implementations, the subject can be undergoing treatments for a disease associated with anti-X antibody (e.g., drug treatments). In some implementations, the subject can be in remission. In some implementations, the remission can be drug-induced. In some implementations, the remission can be drug-free.

In some implementations, the subject can be an animal model for a disease associated with anti-X antibody. In some implementations, the animal model can be a mouse, rabbit or primate model of a disease associated with anti-X antibody. In some implementations, the animal model can involve inducing anti-X antibody responses by immunizing or vaccinating an animal with antigen X.

A subject can be "seronegative for known anti-X antibodies associated with a disease" as determined by the failure to detect the presence of known anti-X antibodies associated with a disease in a blood test.

In some implementations, the known anti-X antibodies associated with a disease can be anti-mitochondrial antibodies (AMA), ANAs, anti-multiple nuclear dots (MND) autoantibodies, anti-nuclear body (NB) autoantibodies, anti-hexokinase 1 (HK1) antibodies and anti-KLHL12 antibodies.

In some implementations, the subject can be asymptomatic. Asymptomatic subjects include healthy subjects who have essentially no risk or only a low risk of developing a disease associated with an anti-X antibody (e.g., there is a less than 10%, less than 5%, less than 3% or less than 1% probability that the asymptomatic patient will develop a disease associated with an anti-X antibody over the following five year period). Asymptomatic subjects further include healthy subjects who have a high risk of developing a disease associated with an anti-X antibody (e.g., there is a greater than 50%, greater than 70%, greater than 90%, or greater than 95% probability that the asymptomatic patient will develop a disease associated with an anti-X antibody over the following five year period). Asymptomatic subjects further include diseased subjects, who can display mild early diagnostic indicators of a disease associated with an anti-X antibody, but who are otherwise disease or complaint free.

It should be noted that the terms "healthy control individual," "healthy subjects," and grammatical equivalents thereof are used interchangeably and refer to subjects who do not have increased anti-X antibody levels above baseline or a standard known or determined to represent subjects who do not have a disease associated with an anti-X antibody.

In some implementations, the healthy subjects can have never suffered from a certain disease. In some implementations, the healthy subjects can be previously diseased. In some implementations, the healthy subjects can be undergoing a routine medical checkup. In some implementations, the healthy subjects can be members of a control group in, for example, a clinical trial. In some implementations, the healthy subjects can be at risk of contracting a disease, as determined by the presence of certain risk factors that are well known in the art. Such risk factors include, without limitation, a genetic predisposition, a personal disease history, a familial disease history, a lifestyle factor, an environmental factor, a diagnostic indicator and the like.

The baseline or standard that determines or defines a subject as a subject not suffering from a disease associated with an anti-X antibody is the reference interval. In diagnostic or health-related fields, the reference interval is a range of values observed in the reference subjects, which can be healthy control individuals, designated by specific percentiles. The reference interval can be any range of values as determined by those having skill in the art. See CLSI, "How to define and determine reference intervals in the clinical laboratory: approved guideline," C28:A2 (2000).

In some cases, the reference interval can be stringent or less stringent depending on the specific analyte being measured or disease being studied. in some implementations, the reference interval can be set at the 95th percentile. In order to increase specificity and decrease sensitivity, e.g., increase stringency, a higher cut-off can be used such as the 96th percentile or the 97th or the 98th or the 99th.

An anti-X antibody can be considered increased in a subject if anti-X antibody levels are at least above the 95th percentile relative to anti-X antibody levels in healthy control subjects. In other implementations, anti-X antibody can be considered increased in a subject if anti-X antibody levels are above the 96th, 97th, 98^{th}, or 99th percentile. A subject with anti-X antibody levels at or above any of the disclosed reference intervals is considered to have a disease associate with anti-X antibody.

In some implementations, the presence of anti-X antibody can be based on a comparison of signal against background in a healthy subject. In some implementations, the presence of an anti-X antibody can be increased or decreased relative to an average or median anti-X antibody level observed in a population of healthy subjects. In some implementations, anti-X antibody can be absent in healthy subjects. In some implementations, an anti-X antibody level cannot be detected above the noise of the respective assay used to determine an anti-X antibody level. In some implementations, an anti-X antibody can be considered present in a sample if an anti-X antibody level can be detected above the noise of the respective assay used to determine an anti-X antibody level. In some implementations, an anti-X antibody can be considered increased in a sample if the signal in an anti-X antibody detection assay is at least two standard deviations above noise such as the average or mean signal for control samples. In some implementations, an anti-X antibody can be considered present in a sample if the level of the anti-X antibody exceeds a predetermined threshold level. An anti-X antibody threshold level can be determined by a skilled artisan, such as a clinical physician, based on a variety of factors, such as the specific objectives of a clinical trial or the diagnostic and prognostic significance of a certain anti-X antibody level or the results of another diagnostic test for a disease associated with an anti-X antibody that does not involve the detection of anti-X antibody levels.

Anti-X antibodies can be detected in a variety of different biological samples obtained from a subject. Examples of "biological sample" include any specimen from the body of an organism that can be used for analysis or diagnosis. As an example, a biological sample can include a liquid sample such as whole blood, plasma, serum, synovial fluid, amniotic fluid, sputum, pleural fluid, peritoneal fluid, central spinal fluid, urine, bile, saliva or tears. A biological sample can also include a solid tissue sample such as a liver biopsy, bone marrow, buccal or other solid or semi-solid aggregated of cells. In the context of the present disclosure, a biological sample obtained from a subject can be any sample that contains or is suspected to contain autoantibodies and encompasses any material from a subject in which an anti-X antibody can be detected.

A biological sample can be or include, for example whole blood, serum, plasma or sputum. In some implementations, a biological sample of the present disclosure can be a tissue biopsy such as a liver biopsy. The liver biopsy can be less than 10 mm, less than 11 mm, less than 12 mm, less than 13 mm, less than 14 mm, less than 15 mm, less than 16 mm, less than 17 mm, less than 18 mm, less than 19 mm, less than 20 mm, less than 21 mm, less than 22 mm, less than 21 mm, less than 23 mm, less than 24 mm or less than 25 mm in length. The liver biopsy can include, in addition to the lengths disclosed herein, at least 4 portal triads, at least 5 portal triads, at least 6 portal triads, at least 7 portal triads, at least 8 portal triads, at least 9 portal triads, at least 10 portal triads or at least 11 portal triads.

A biological sample can be obtained from any subject that contains or is suspected to contain anti-X antibodies. A biological sample can also be obtained from any subject that does not or is not suspected to have anti-X antibodies. Thus, in some implementations, a biological sample can be obtained from a symptomatic subject, an asymptomatic subject and a subject that is negative for anti-X antibodies. In some implementations, the biological sample can be obtained from a mammal.

A biological sample can be collected and processed immediately or it can be collected, frozen and processed at a later date. The biological sample could also include a plurality of samples from one of the subjects described herein. In some implementations, the plurality of biological samples can be collected periodically over the course of more than 12 hours, more than 1 day, more than 2 days, more than 3 days, more than 4 days, more than 5 days, more than 6 days, more than 7 days, more than 10 days, more than 14 days, more than 3 weeks, more than 1 month, more than 2 months, more than 3 months, more than 4 months, more than 5 months, more than 6 months, more than 9 months, more than 12 months, more than 18 months, more than 24 months, more than 30 months, more than 3 years months, more than 4 years, or more than 5 years.

In some implementations, the example techniques described herein can be performed by contacting the biological sample with 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, or 16 or more of the support-antigen complexes described herein. Two or more antigens can be conjugated to a single solid support as disclosed herein.

The detection of the presence of the bound anti-X antibodies may include contacting anti-X antibodies with a detection probe (e.g., an antigen) specific to anti-X antibodies and detecting specific binding of the antibody to the detection probe.

A reporter tag may be used to detect an antibody. Reporter tags function to produce a signal for detection of a biomarker. In some implementations, reporter tag includes a ligand or particle. In some implementations, the ligand is biotin. In some implementations, the particle includes a nanoparticle. Reporter tags can be attached, for example, to any of the detection probes used herein through non-covalent or covalent cross-linkage. A reporter tag can include a label of the type described herein. For example, a label can include a fluorophore, an enzyme, a chemiluminescent moiety, a radioactive moiety, an organic dye, a small molecule, a polypeptide or functional fragment thereof. In some implementations, a label includes a fluorescent label. In some implementations, the fluorescent label is PE, horseradish peroxidase or alkaline phosphatase. In some implementations, a label is conjugated to a detection probe of the disclosure as exemplified above.

As provided herein, a disease associated with anti-X antibody can be determined in a subject by detecting the presence of the bound anti-X antibodies. Techniques for detecting, measuring and/or quantifying a signal produced by a label of the present disclosure are well known in the art and include detection of fluorescence, luminescence, chemiluminescence or absorbance, reflectance, transmittance, birefringence or refractive index. Optical techniques include imaging techniques such as confocal and non-confocal microscopy and non-imaging techniques such as microplate readers. In some implementations, techniques of detecting anti-X antibody in biological sample can include visualization, quantification or both of a fluorescent, colorimetric or absorbance signal in a biological sample.

Elements of different implementations described may be combined to form other implementations not specifically set forth previously. Elements may be left out of the systems described previously without adversely affecting their operation or the operation of the system in general. Furthermore, various separate elements may be combined into one or more individual elements to perform the functions described in this specification.

Other implementations not specifically described in this specification are also within the scope of the following claims.

## Claims

1. A complex comprising:
a solid support; and
at least two different types of antigens attached to the solid support.

2. The complex of claim 1, further comprising:
a carrier attached to the solid support;
wherein at least one of the different types of antigens is attached to the carrier, the at least one of the different types of antigens being attached to the solid support through the carrier.

3. The complex of claim 2, further comprising:
a linker to attach the at least one of the different types of antigens to the carrier.

4. The complex of claim 3, wherein the linker comprises a polyethylene glycol (PEG) spacer or biotin.

5. The complex of claim 2, wherein the carrier comprises at least one of streptavidin, a polymerized protein, polymerized streptavidin with biotin, bovine serum albumin (BSA), human serum albumin (HSA), polyethylene glycol (PEG) spacer attachment, or adipic acid dihydrazide (ADH).

6. The complex of claim 1, further comprising:
multiple carriers, each of the carriers being attached to the solid support;
wherein each of the different types of antigens is attached to a respective one of the multiple carriers, each of the different types of antigens being attached to the solid support through the respective one of the multiple carriers.

7. The complex of claim 1, further comprising:
a carrier attached to the solid support;
wherein each of the different types of antigens is attached to the carrier, each of the different types of antigens being attached to the solid support through the carrier.

8. The complex of claim 1, wherein the at least two different types of antigens comprise a group of markers for a disease or condition of a subject

9. The complex of claim 1, wherein at least one of the different types of antigens is part of a cellular extract that is attached to the solid support.

10. The complex of claim 1, wherein a first one of the different types of antigens comprises a purified antigen and a second one of the different types of antigens is part of a cellular extract.

11. The complex of claim 1, wherein the at least two different types of antigens comprise at least two of the following: Scl-70, centromere, dsDNA, Ro52, Ro60, SS-B, Ribo-P, DFS-70, EJ, Sp100, Gp210, actin, AMA-M2, AMA-M2/MIT3, TIF1-γ, NXP-2, SAE, PL-7, PL-12, OJ, HMGCR, SRP, Mi-2, MDA-5, RNA Helicase, Ki/SL, Sm/RNP, SS-A, PM/Scl, RNA Pol III, Th/To, Fibrillarin, Sm, Ku, Jo-1, RNP, BICD2, HK, KL, LC1, LKM-1, SLA, SS-A/Ro52, Scl-70, Ku, Centromere proteins, U11/U12 RNP, Hexokinase-1, or Kelch-12.

12. The complex of claim 1, where at least one of the different types of antigens binds to an antinuclear antibody (ANA).

13. The complex of claim 1, further comprising:
a label associated with the solid support, the label being associated with a disease, condition, or analyte that is detectable using the at least two different antigens.

14. A kit comprising:
a first solid support comprising first antigens attached thereto, the first antigens comprising at least two different types of antigens; and
a second solid support comprising at least one second antigen attached thereto, the at least one second antigen being different than each of the first antigens;
wherein one or more of the first antigens or the at least one second antigens comprises a purified antigen or is part of a cellular extract.

15. The kit of claim 14, wherein the first solid support comprises a first label and the second solid support comprises a second label, the first label being different from the second label.

16. The kit of claim 14, wherein the first antigens comprise markers for a first disease or condition, the first label being associated with the first disease or condition; and
wherein the at least one second antigen comprises a marker for a second disease or condition, the second label being associated with the second disease or condition.

17. The kit of claim 14, wherein the first antigens comprise first labels that produce a signal in response to binding with corresponding antibodies; and
wherein the at least one second antigen comprises at least one second label that produces a signal in response to binding with at least one corresponding antibody;
wherein the first labels and the at least one second label are of a same type or of a different type.

18. A method comprising:
contacting a biological sample from a subject with a complex comprising:
a solid support; and
at least two different types of antigens attached to the solid support; and
detecting a presence of an antibody in the biological sample bound to the complex, where the presence of the antibody bound to the complex is indicative of a disease.

19. The method of claim 18, further comprising:
detecting the solid support based on a label on the solid support, the label being associated the disease;
wherein detecting comprises detecting signals from labels on the at least two different types of antigens.

20. The method of claim 18, wherein the complex is one of multiple complexes that contacts the biological sample, each of the multiple complexes having a different configuration associated with a different disease, each of the multiple complexes comprising:
a respective solid support; and
one or more antigens attached to the respective solid support.
